# EUROPÄISCHE PATENTANMELDUNG

(11) **EP 3 470 847 A2**
(43) Veröffentlichungstag der Anmeldung: **17.04.2019**
(21) Anmeldenummer: 18206804.9
(22) Anmeldetag: 21.12.2012
(51) Int. Cl.: G01N 33/68, C09J 201/00

(54) **STANDARD ZUR QUANTIFIZIERUNG VON PATHOGENEN AGGREGATEN AUS KÖRPEREIGENEN PROTEINEN**

(30) Priorität: 23.12.2011 DE 102011057019
(62) Teilanmeldung aus: 12816051.2
(71) Anmelder: Forschungszentrum Jülich GmbH, 52425 Jülich (DE)
(72) Erfinder: Willbold, Dieter, 52425 Jülich (DE); Funke, Susanne Aileen, 96242 Sonnefeld (DE)
(74) Vertreter: Fitzner, Uwe

(57) **Zusammenfassung**

Die Erfindung betrifft Standards zur Quantifizierung von pathogenen Aggregaten oder Oligomeren aus körpereigenen Proteinen, die eine Proteinaggregationserkrankung, eine amyloide Degeneration oder Proteinfehlfaltungserkrankungen kennzeichnen sowie die Verwendung dieser Standards für die Quantifizierung dieser pathogenen Aggregate oder Oligomere.

## Beschreibung

Die Erfindung betrifft Standards zur Quantifizierung von pathogenen Aggregaten oder Oligomeren aus körpereigenen Proteinen, die eine Proteinaggregationserkrankung, eine amyloide Degeneration oder Proteinfehlfaltungserkrankungen kennzeichnen sowie die Verwendung dieser Standards für die Quantifizierung dieser pathogenen Aggregate oder Oligomere.

Als Proteinfehlfaltungserkrankungen bzw. Proteinaggregationserkrankungen oder amyloide Degeneration wird eine heterogene Gruppe klinischer Zustände bezeichnet, deren gemeinsames Kriterium in vielen Fällen (aber nicht ausschließlich) die extrazelluläre systemische oder lokale Ablagerung eines jeweils spezifischen Proteins in der geordneten Konformation einer Beta-Faltblattstruktur ist. Unter diese Gruppe fällt auch die Alzheimer-Krankheit (AD, Alzheimersche Demenz, lateinisch = Morbus Alzheimer) oder Morbus Parkinson. Die altersbedingte Demenz stellt in der heutigen Gesellschaft ein immer größeres Problem dar, da durch die gestiegene Lebenserwartung immer mehr Menschen davon betroffen sind und die Krankheit sich somit auf die sozialen Sicherungssysteme und deren Finanzierbarkeit auswirkt.

Pathologische Aggregate aus körpereigenen Proteinen, wie z.B. Oligomere oder Fibrillen, treten in vielen neurodegenerativen Erkrankungen auf. Bei der Alzheimerschen Demenz findet man z.B. im Gehirn Amyloid-Beta-Peptid-Ablagerungen (A-Beta-Peptid-Ablagerungen) und bei Morbus Parkinson Synuclein-Ablagerungen. Die Amyloid-Beta-Peptid-Ablagerungen (oder Plaques, bestehend aus Peptid-Fibrillen) stellen allerdings lediglich das Endstadium eines Prozesses dar, der mit der Abspaltung von monomeren Amyloid-Beta-Peptiden aus APP (Amyloid Precurser Protein) beginnt, anschließend neurotoxische Amyloid-Beta-Peptid-Oligomere ausbildet und schließlich mit der Ablagerung von Amyloid-Beta-Peptid-Fibrillen in Plaques endet. Pathologisches Hauptmerkmal der AD ist die Bildung von senilen oder amyloiden Plaques, bestehend aus dem A-Beta-Peptid. Des Weiteren entstehen neurofibrilläre Ablagerungen aus dem Tau-Protein. Das Vorläufer-Protein des A-Beta-Peptids, das APP, ist in der Zellwand der Neuronen lokalisiert. Durch proteolytischen Abbau und gegebenenfalls nachträgliche Modifikation entstehen daraus A-Beta-Fragmente unterschiedlicher Länge und Art, wie z.B. A-Beta 1-40, A-Beta 11-40, A-Beta 1-42, A-Beta 11-42 oder pyroGluA-Beta 3-42, pyroGluA-Beta 3-40. Monomere A-Beta-Peptide entstehen während des gesamten Lebens auch im gesunden Organismus.
Gemäß der Amyloid-Kaskaden-Hypothese aus den 1990er Jahren sind die A-Beta-Ablagerungen in Form von Plaques die Auslöser der Krankheitssymptome. In den letzten Jahren weisen jedoch unterschiedliche Studien darauf hin, dass besonders die kleinen, frei diffundierenden A-Beta-Oligomere die größte Toxizität besitzen, und für die Entstehung und den Fortschritt der AD verantwortlich sind. Somit sind Aggregate des A-Beta-Peptids unmittelbar mit der AD-Pathogenese verknüpft.
Eine sichere Diagnose ist heutzutage allerdings erst nach Auftreten von auffälligen, klinischen Symptomen möglich, man geht dabei von einer Zuverlässigkeit von maximal 90 % aus. Einzige bisher sichere Diagnosemöglichkeit besteht z.Z. erst nach dem Tod des Patienten durch histologischen Nachweis unterschiedlicher Veränderungen im Gehirn.

Demgemäß besteht ein Bedarf an Verfahren zur Identifizierung und quantitativer Erfassung von pathologischen Aggregaten oder Oligomeren aus körpereigenen Proteinen, die eine Proteinaggregationserkrankung, eine amyloide Degeneration oder eine Proteinfaltungserkrankung hervorrufen und/oder kennzeichnen.

Bis heute sind nur wenige Methoden zur Charakterisierung und Quantifizierung von pathogenen Aggregaten oder Oligomeren aus körpereigenen Proteinen, die eine Proteinaggregationserkrankung, eine amyloide Degeneration oder Proteinfehlfaltungserkrankung hervorrufen, in Geweben und Körperflüssigkeiten beschrieben worden.

Für die Entwicklung solcher Methoden, sowie um die Vergleichbarkeit der damit bestimmten Ergebnisse zu gewährleisten, sind genau definierte Standards, d.h. genau charakterisierte (synthetische) Polymere notwendig. Für den Einsatz als Standard müssen diese in verschiedenen Größen und Formen vorliegen, die jedoch genau definiert sein müssen.

Die Aggregation von Peptiden wird jedoch von einer Vielzahl von Faktoren bestimmt, wie z.B. Temperatur, Salzgehalt der Probe, Herstellerfirma der Proteine, Reinheit usw.. Dadurch ist die Herstellung von Polymeren als Standards durch Aggregation von Monomer-Peptiden für die Testentwicklung und Validierung schwer reproduzierbar.
Bisher waren außerdem z.B. präparierte A-Beta-Oligomere nicht stabil genug, d.h. es konnte nicht gewährleistet werden, dass bei der Entnahme von A-Beta-Oligomeren aus einer Präparation zu verschiedenen Zeitpunkten immer die gleichen A-Beta-Aggregatspezies vorlagen.
Die bisher bekannten Oligomerpräparationen bestehen in der Regel aus verschiedenen Intermediatformen, die uneinheitlich groß sind, und dadurch unzureichend reproduzierbar sind.

Es wurde jedoch eine Reihe von Verbindungen beschrieben, die an anti-Amyloidmonoklonale Antikörper binden. Solche Verbindungen sind zum Beispiel aus Manea et al., (Biopolymeres Peptide Science 2004, Vol. 76, S. 503 - 511 und Peptid-Science 2008, Vol. 90, No. 2, S. 94 - 104) sowie Chafekar et al. (ChemBioChem 2007, 8, 1857 - 1864) bekannt. Diese basieren auf Polymeren, an die A-beta-Epitope (4-10) oder (16-20) gebunden sind. Ein Verfahren zum Nachweis von Beta-Amyloid-Peptiden ist aus der US 5,593,846 bekannt, wobei Antikörper eingesetzt werden, die an die Positionen 13-28 sowie 1-16 des Peptids binden. Allerdings werden hier nicht ausschließlich Aß-Oligomere detektiert, sondern auch Monomere.

Insbesondere im Bereich des Nachweises von A-Beta-Oligomeren in Proben aus Gewebe oder Körperflüssigkeiten wurden bisher als Vergleichswert A-Beta-Oligomere eingesetzt, die aus synthetisch hergestellten A-Beta-Monomeren mittels verschiedener Protokolle präpariert wurden. Es war nicht gewährleistet, dass in den Oligomerpräparationen nur eine Oligomergröße vorlag, und die Präparationen keine A-Beta-Monomere oder -Fibrillen enthielten.
Ferner zeigten diese Proben keine ausreichende Lagerstabilität und veränderten im Bezug auf ihre Oligomer-Monomer-Zusammensetzung ihre Eigenschaften.

Aufgrund dieser Nachteile war bisher eine exakte Kalibrierung und Charakterisierung von Oligomernachweissystemen ungenau oder eher unmöglich. Insbesondere durch die unterschiedlichen Protokolle in den verschiedenen Labors war eine weltweite Harmonisierung und damit Etablierung von Testsystemen erschwert.

Es besteht somit ein Bedarf an Standards zur Quantifizierung von pathogenen Aggregaten oder Oligomeren aus körpereigenen Proteinen, die eine Proteinfehlfaltungserkrankung, amyloide Degeneration oder Proteinaggregationserkrankung hervorrufen und/oder kennzeichnen.

Aufgabe der vorliegenden Erfindung war es, Standards zur Verfügung zu stellen, die eine exakte und quantitative Bestimmung von pathogenen Aggregaten oder Oligomeren aus körpereigenen Proteinen möglich machen.
Die Standards sollen als interne oder externe Standards einsetzbar sein.

Ferner war es Aufgabe der vorliegenden Erfindung, genau definierte, homogene und stabile Präparationen von Standards zur Quantifizierung von pathogenen Aggregaten oder Oligomeren aus körpereigenen Proteinen zur Verfügung zu stellen.

Gelöst wird diese Aufgabe durch Standards zur Quantifizierung von Oligomeren oder pathogenen Aggregaten, die eine Proteinaggregationserkrankung oder eine amyloide Degeneration oder Proteinfehlfaltungserkrankung kennzeichnen, dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Als Standard im Sinne der vorliegenden Erfindung wird eine allgemein gültige und akzeptierte, feststehende Bezugsgröße bezeichnet, die zum Vergleichen und Bestimmen von Eigenschaften und/oder Menge dient, insbesondere zur Bestimmung der Größe und Menge von pathogenen Aggregaten aus körpereigenen Proteinen. Der Standard im Sinne der vorliegenden Erfindung kann zum Kalibrieren von Geräten und/oder Messungen verwendet werden.

Im Sinne der vorliegenden Erfindung können unter dem Begriff "Proteinaggregationserkrankung" auch amyloide Degenerationen und Proteinfehlfaltungserkrankungen zusammengefasst werden. Beispiele solcher Krankheiten und die damit verbundenen körpereigenen Proteine sind: A-Beta- und Tau-Protein für AD, alpha-Synuclein für Parkinson oder Prion-Protein für Prion-Erkrankungen, zum Beispiel wie die humane Creutzfeld-Jakob-Krankheit (CJD), die Schafskrankheit Scrapie und die bovine spongiforme Enzephalopatie (BSE).

"Homologe Sequenzen" bedeutet im Sinne der Erfindung, dass eine Aminosäuresequenz eine Identität mit einer Aminosäuresequenz aus einem körpereigenem pathogenen Aggregat oder Oligomeren, das eine Proteinaggregationserkrankung hervorruft, von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch wenn sie dieselbe Aminosäuresequenz besitzen.

Unter dem Begriff "entsprechender Teilbereich" körpereigener Proteine ist jene Peptidsequenz zu verstehen, die gemäß den erfindungsgemäßen Definitionen eine identische oder mit der angegebenen Prozentzahl homologe Peptidsequenz eines Monomers, aus dem die erfindungsgemäßen Standards aufgebaut werden, aufweist.

Wesentlich für die erfindungsgemäßen Standards ist, dass die Standards nicht aggregieren, bevorzugt durch die Verwendung von monomeren Sequenzen, die nicht aggregieren, da der "entsprechender Teilbereich" körpereigener Proteine für die Aggregation nicht verantwortlich ist, oder die durch Blockierung der für die Aggregation verantwortlichen Gruppen nicht aggregieren.

Aggregate im Sinne der vorliegenden Erfindung sind
- Partikel, die aus mehreren, bevorzugt gleichen Bausteinen bestehen, die nicht kovalent miteinander verbunden sind und/oder
- nicht-kovalente Zusammenlagerungen mehrerer Monomere.

In einer Ausführung der vorliegenden Erfindung besitzen die Standards eine genau definierte Anzahl von Epitopen, die kovalent miteinander verknüpft sind (unmittelbar oder über Aminosäuren, Spacer und/oder funktionelle Gruppen) für die Bindung der entsprechenden Sonden.
Sonden im Sinne der Erfindung werden ausgewählt aus der Gruppe bestehend aus: Antikörper, Nanobody und Affibody. Sonden sind darüber hinaus alle Moleküle, die eine hinreichende Bindespezifität für das zu detektierende Aggregat besitzen, z.B. Farbstoffe (ThioflavinT, Kongorot, etc.).

Die Zahl der Epitope wird dadurch bestimmt, dass eine Polypeptid-Sequenz verwendet wird, die bezüglich ihrer Sequenz identisch mit jenem Teilbereich der körpereigenen Proteine ist, die ein Epitop bildet bzw. eine Homologie von mindestens 50 % mit diesem Teilbereich aufweist, und dabei die biologische Aktivität des Epitopes besitzt. Eine so ausgewählte Polypeptid-Sequenz wird in der gewünschten Anzahl bei dem Aufbau der erfindungsgemäßen Standards eingebaut und/oder miteinander erfindungsgemäß verknüpft.

Die erfindungsgemäßen Standards sind Polymere, die aus den oben beschriebenen Polypeptid-Sequenzen, bevorzugt Epitope aufgebaut sind, gegebenenfalls enthaltend weitere Elemente.
In einer weiteren Ausführung der vorliegenden Erfindung stellen oben beschriebenen Polypeptid-Sequenzen, bevorzugt Epitope, und/oder deren Homologe mit der biologischen Aktivität des entsprechenden Epitops, die gleiche oder größte Anzahl an Monomeren bezogen auf die Anzahl jeweils einer der restlichen Monomer-Arten des Standards und/oder bezogen auf die Anzahl aller anderen Monomere.

In einer weiteren Ausführung der vorliegenden Erfindung handelt es sich bei den Epitopen um Epitope des A-Beta-Peptids, ausgewählt aus den Teilbereichen A-Beta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5) und pyroGluA-Beta 3-11 (SEQ ID NO: 6), A-Beta 11 - 16 (SEQ ID NO: 7) und pyroGluA-Beta 11-16 (SEQ ID NO: 8), zum Beispiel des humanen N-terminale Epitops (mit folgender Sequenz: DAEFRHDSGYE (1-11; entspricht SEQ ID NO: 3).
PyroGlu ist die Abkürzung für ein Pyroglutamat, welches sich aus dem Glutamat-Rest an Position 3 oder 11 des A-beta-Peptids bilden kann, nachdem die N-terminal davon liegenden Reste abgespalten wurden.

Das erfindungsgemäße Standardmolekül ist ein Polymer aus den oben definierten Polypeptid-Sequenzen. Unter Oligomer im Sinne der Erfindung ist ein Polymer aus 2, 3, 4, 5, 6, 7, 8, 9, 10, 11, 12, 13, 14, 15, 16, 17, 18, 19 oder 20 Monomeren (unter Monomer ist die o.g. Polypeptid-Sequenz zu verstehen) gebildet, oder Vielfache davon, bevorzugt 2 - 16, 4-16, 8-16, besonders bevorzugt 8 oder 16, oder Vielfache davon.
Bei den erfindungsgemäßen Standards handelt es sich also um erfindungsgemäße Oligomere beziehungsweise Polymere.

In einer Alternative der vorliegenden Erfindung sind die Standards wasserlöslich.

In einer Alternative der vorliegenden Erfindung sind die erfindungsgemäßen Standards aus gleichen Polypeptid-Sequenzen aufgebaut.
In einer Alternative der vorliegenden Erfindung sind die erfindungsgemäßen Standards aus unterschiedlichen Polypeptid-Sequenzen aufgebaut.

In einer Alternative der vorliegenden Erfindung werden solche oben definierte Polypeptid-Sequenzen in einer linearen-Konformation aneinandergereiht.
In einer Alternative der vorliegenden Erfindung werden solche oben definierte Polypeptid-Sequenzen zu einem verzweigten, erfindungsgemäßen Oligomer aneinandergereiht.
In einer Alternative der vorliegenden Erfindung werden solche oben definierte Polypeptid-Sequenzen zu einem cross-linked, erfindungsgemäßen Oligomer aneinandergereiht.
Verzweigte oder cross-linked, erfindungsgemäße Oligomere können durch Verknüpfung einzelner Bausteine mittels Lysin oder mittels Click-Chemie hergestellt werden.
Wie oben beschrieben, können die erfindungsgemäßen Standards, also die erfindungsgemäßen Oligomere beziehungsweise Polymere, zusätzlich zu den in genau definierter Anzahl vorliegenden Polypeptid-Sequenzen, bevorzugt Epitopen, noch zusätzliche Aminosäuren, Spacer und/oder funktionelle Gruppen enthalten, über welche die Polypeptid-Sequenzen, bevorzugt Epitope, kovalent miteinander verknüpft sind.
In einer Alternative ist die unmittelbare Verknüpfung der Polypeptid-Sequenzen, bevorzugt Epitope mit Cystein, insbesondere mittels Disulfid-Verbrückung durch Cysteine ausgeschlossen (um zu vermeiden, dass reduzierende Agenzien die Verbrückung lösen). Ebenso ist in einer weiteren Variante eine unmittelbare Verknüpfung der Spacer mit der Polypeptid-Sequenz einerseits und mit Cystein andererseits ausgeschlossen.

Die Erfindung betrifft in einer Alternative ein Standardmolekül, enthaltend oder aufgebaut aus Kopien des aminoterminalen Teils des A-Beta-Peptids, ausgewählt aus den Teilbereichen A-Beta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5) und pyroGluA-Beta 3-11 (SEQ ID NO: 6), A-Beta 11 - 16 (SEQ ID NO: 7) und pyroGluA-Beta 11-16 (SEQ ID NO: 8)zum Beispiel des humanen N-terminale Epitops (mit folgender Sequenz: DAEFRHDSGYE (1-11).

Die Vervielfältigung der Epitope durch funktionelle Gruppen kann vor oder nach der Synthese der einzelnen Bausteine durchgeführt werden. Charakteristisch für die erfindungsgemäßen Standards ist die kovalente Verknüpfung der Polypeptid-Sequenzen.

Die erfindungsgemäß einzusetzenden Polypeptid-Sequenzen können identisch mit der Sequenz des A-Beta-Volllängen-Peptids sein oder zeigen eine Homologie von 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % mit der Sequenz des A-Beta-Volllängen-Peptids.

Alternativ werden auch zum Aufbau der erfindungsgemäßen Standard-Moleküle Polypeptid-Sequenzen eingesetzt, die identisch mit einem Teilbereich des A-Beta-Volllängen-Peptids sind, bzw. eine Homologie von 50, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % mit einem Teilbereich des A-Beta-Volllängen-Peptids zeigen. Wesentlich für die erfindungsgemäß eingesetzten Sequenzen ist ihre Eigenschaft nicht (oder nur gemäß der Bedingungen kontrolliert) zu aggregieren und/oder ihre die Aktivität als Epitop.

In einer weiteren Ausführung der vorliegenden Erfindung sind die Standards als Dendrimere aufgebaut. Die erfindungsgemäßen Dendrimere sind aus den oben beschriebenen erfindungsgemäß zu verwendenden Polypeptid-Sequenzen aufgebaut und können ein zentrales Gerüstmolekül enthalten. Bevorzugt ist das Gerüstmolekül ein Streptavidin-Monomer, besonders bevorzugt ein Polymer, insbesondere Tetramer.

Die erfindungsgemäßen Dendrimere enthalten in einer Variante Polypeptid-Sequenzen, die eine Sequenz besitzen, die identisch mit einem Teilbereich des A-Beta-Peptids ist, oder eine mindestens 50 %ige Homologie zu dem entsprechenden Teilbereich zeigt.

Erfindungsgemäß ist unter dem Begriff mindestens 50 %ige Homologie auch eine höhere Homologie zu verstehen, ausgewählt aus der Gruppe bestehend aus 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % zu verstehen.

Standards, vorteilhaft mit höherer Löslichkeit im Wässrigen als pathogene Aggregate oder Oligomere aus körpereigenen Proteinen, sind in einer Ausführung der Erfindung aus Polypeptid-Sequenzen gebildet, die identisch mit dem N-terminalen Bereich des A-Beta-Peptids sind oder mindestens 50 %ige Homologie dazu aufweisen. Erfindungsgemäß ist unter dem N-terminalen Bereich von einem A-Beta-Polypeptid die Aminosäuresequenz A-Beta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5) und pyroGluA-Beta 3-11 (SEQ ID NO: 6), A-Beta 11 - 16 (SEQ ID NO: 7) und pyroGluA-Beta 11-16 (SEQ ID NO: 8) zu verstehen.

Ein erfindungsgemäßes Standardmolekül kann Epitope für mindestens 2, 3, 4, 5, 6, 7, 8, 9, 10 oder mehr verschiedene Sonden enthalten.

Epitope, charakteristisch für verschiedene Sonden, können in die erfindungsgemäßen Standards dadurch eingebaut werden, dass Polypeptid-Sequenzen verwendet werden, die identisch mit unterschiedlichen Bereichen des A-Beta-Peptids sind, bzw. mindestens eine 50 %ige Homologie dazu aufweisen, aber die Aktivität des entsprechenden Epitops besitzen.
In einer Ausführung werden hierzu Polypeptidsequenzen eingesetzt, die identisch oder eine 50 %ige Homologie mit dem N-terminalen Bereich des A-Beta-Polypeptids aufweisen, sowie Polypeptid-Sequenzen, die identisch bzw. mindestens eine 50 %ige Homologie mit dem C-Terminus des A-Beta-Polypeptids aufweisen.
In einer Ausführung der vorliegenden Erfindung enthalten die Standardmoleküle sog. Spacer.
Unter einem Spacer ist ein Molekül zu verstehen, das über kovalente Bindungen in das Standardmolekül eingebaut ist, und bestimmte physikalische und/oder chemische Eigenschaften besitzt, durch welche die Eigenschaften des Standardmoleküls verändert werden. In einer Ausführung der erfindungsgemäßen Standards werden hydrophile oder hydrophobe, bevorzugt hydrophile Spacer, eingesetzt. Hydrophile Spacer werden ausgewählt aus der Gruppe der Moleküle, gebildet aus Polyethylenglycol, Zucker, Glycerin, Poly-L-Lysin oder beta-Alanin.

Die erfindungsgemäßen Standards enthalten in einer Alternative der vorliegenden Erfindung (weitere) funktionelle Gruppen.
Unter funktionellen Gruppen sind Moleküle zu verstehen, die kovalent an die Standardmoleküle gebunden sind. In einer Variante enthalten die funktionellen Gruppen Biotin-Gruppen. Dadurch wird eine starke kovalente Bindung an Streptavidin ermöglicht. Standardmoleküle enthaltend Biotin-Gruppen können so an Moleküle, enthaltend Streptavidin-Gruppen, gebunden werden. Falls die erfindungsgemäßen Standardmoleküle Biotin und/oder Streptavidin-Gruppen enthalten, können so größere Standards zusammengebaut werden oder mehrere, ggf. unterschiedliche Standardmoleküle, an ein Gerüst gebunden werden.

In einer weiteren Alternative der vorliegenden Erfindung enthalten die Standardmoleküle Farbstoffe zur spektralphotometrischen Bestimmung und/oder aromatische Aminosäuren. Aromatische Aminosäuren sind z.B. Tryptophane, Tyrosin, Phenylalanin oder Histidin, bzw. ausgewählt aus dieser Gruppe. Durch den Einbau von Tryptophan wird eine spektralphotometische Bestimmung der Konzentration von Standards in Lösung ermöglicht.

Weiterer Gegenstand der vorliegenden Erfindung sind Dendrimere enthaltend Polypeptide, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung kennzeichnen.

Die erfindungsgemäßen Dendrimere können jede der oben beschriebenen Merkmale der Standards oder jede beliebige Kombination davon enthalten.

In einer Alternative der vorliegenden Erfindung handelt es sich um:
Dendrimere enthaltend eine genau definierte Anzahl von Epitopen für die kovalente Bindung von Sonden,
Dendrimer enthaltend Epitope des A-beta-Peptids,
Dendrimer dadurch gekennzeichnet, dass es eine höhere Löslichkeit im Wässrigen besitzt, als die pathogenen Aggregate aus körpereigenen Proteinen, die eine Proteinaggregationserkrankung kennzeichnen,
Dendrimer enthaltend funktionelle Gruppen,
Dendrimer enthaltend mindestens ein Spacer-Molekül und/oder
Dendrimer enthaltend Farbstoffe zur spektralphotometrischen Bestimmung und/oder aromatische Aminosäuren.

Erfindungsgemäß haben die Dendrimere eine radiale Symmetrie.
In einer Veriante erfolgt die Verzweigung der ersten Generation des Dendrimers über Lysin, inbesondere drei Lysin-Aminosäuren.

In einer weiteren Alternative der vorliegenden Erfindung sind in den Standards, insbesondere Dendrimeren, die Polypeptid-Sequenzen, bevorzugt Epitope, nicht über eine Bindung zu einem Schwefelatom, nicht über eine Thioether-Bindung und/oder nicht über Cystein (gegebenenfalls mittels Disulfid-Verbrückung durch Cystein) miteinander oder mit anderen Elementen der Standards wie Aminosäuren, Spacer und/oder funktionelle Gruppen und/oder andere oben beschriebenen Elementen verknüpft, insbesondere kovalent gebunden. Ebenso sind in einer weiteren Variante die Polypeptid-Sequenzen, bevorzugt Epitope, und ein daran gebundener Spacer am Spacer nicht über eine Bindung zu einem Schwefelatom, nicht über eine Thioether-Bindung und/oder nicht über Cystein miteinander oder mit anderen Elementen der Standards wie Aminosäuren, weitere Spacer und/oder funktionelle Gruppen und/oder andere oben beschriebenen Elementen verknüpft, insbesondere kovalent gebunden.

Die vorliegende Erfindung betrifft ferner ein Verfahren zur Herstellung eines Standards, wie oben beschrieben.
In einer Ausführung wird der erfindungsgemäße Standard mittels Peptidsynthese oder rekombinanter Verfahren hergestellt, die dem Fachmann bekannt sind.

Ein weiterer Gegenstand der vorliegenden Erfindung ist die Verwendung eines oben beschriebenen Standards oder eines oben beschriebenen Dendrimers zur Quantifizierung von pathogenen Aggregaten oder Oligomeren aus körpereigenen Proteinen, die eine Proteinaggregationserkrankung kennzeichnen.

In einer Ausführung der Erfindung wird der Standard verwendet um A-beta-Oligomere zu quantifizieren.

Gegenstand der vorliegenden Erfindung ist somit auch ein Verfahren zur Quantifizierung von von pathogenen Aggregaten oder Oligomeren aus körpereigenen Proteinen, die eine Proteinaggregationserkrankung oder eine amyloide Degeneration oder Proteinfehlfaltungserkrankung kennzeichnen, bei dem die erfindungsgemäßen Oligomere beziehungsweise Polymere als Standard eingesetzt werden.

Die erfindungsgemäßen Standards werden in einer Ausführung der vorliegenden Erfindung für die Kalibrierung bei der Surface-FIDA-Methode, Elisa (Sandwich-Elisa) oder FACS eingesetzt.

In einer anderen Ausführungsform betrifft die vorliegende Erfindung ein Kit, welches erfindungsgemäßen Standard umfasst. Die Verbindungen und/oder Komponenten des Kits der vorliegenden Erfindung können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotitterplatte, absorbiert sein. Ferner kann das Kit Anweisungen für den Gebrauch des Kits für eine Beliebige der Ausführungsformen enthalten.

In einer Alternative der vorliegenden Erfindung werden die Standards zur Quantifizierung von pathogenen Aggregaten oder Oligomeren aus körpereigenen Proteinen verwendet indem:
in einem ersten Schritt die Standards oder die Dendrimere mit Sonden markiert werden und die Anzahl der an die Standards oder Dendrimere gebundenen Sonde bestimmt wird,
in einem zweiten Schritt pathogene Aggregaten oder Oligomere aus körpereigenen Proteinen, die eine Proteinaggregationserkrankung kennzeichnen, mit Sonden markiert werden, die Zahl der an jeweils ein pathogenes Aggregat oder Oligomer bindende Sonden bestimmt wird,
in einem dritten Schritt die Zahl der an jeweils ein Standard oder Dendrimer bindende Sonden aus Schritt 1 mit der aus Schritt 2 verglichen wird, und
in einem vierten Schritt dadurch die Zahl und die Größe der Oligomere aus der Körperflüssigkeit bestimmt wird.

In einer Variante der vorliegenden Erfindung werden die erfindungsgemäßen Standards, bevorzugt Dendrimere, für die Kalibrierung der Surface-FIDA-Methode eingesetzt. In einem ersten Schritt werden körpereigene pathogene Aggregate aus Körperflüssigkeiten, z.B. A-beta-Aggregate, durch ein Fängermolekül, z.B. Capture-Antikörper, auf eine Glasfläche immobilisiert. Im Falle von A-beta-Aggregaten kann hierzu ein N-terminal bindender Capture-Antikörper eingesetzt werden. Nach der Immobilisierung werden die Aggregate durch zwei unterschiedliche Sonden markiert. Im Falle von A-beta-Aggregaten werden z.B. A-beta-Antikörper eingesetzt, die beide über N-terminale Bindeepitope gebunden werden. Die Detektionssonden sind mit, bevorzugt unterschiedlichen, Fluoreszenzfarbstoffen markiert. Dadurch werden sie im Mikroskop, z.B. Laserscanningmikroskop sichtbar.
Erfindungsgemäß wird eine Monomer-Detektion von körpereigenen Polypeptiden ausgeschlossen, indem im Testsystem drei unterschiedliche bzw. drei unterschiedlich markierte Sonden eingesetzt werden, die an einem ähnlichen bzw. gleichen Epitop binden. Alternativ oder zusätzlich kann die Detektion von Monomeren ausgeschlossen werden, indem Signale mit einer niedrigeren Intensität durch die Definition eines Intensität-Schwellenwerts nicht gewertet werden. Da größere Aggregate mehrere Bindungsstellen für die beiden mit unterschiedlichen markierten Farbstoffen Sonde besitzen, kann eine Monomer-Detektion alternativ oder zusätzlich durch Kreuzkorelation dieser Signale ausgeschlossen werden.
Die erfindungsgemäßen Standards können als interne oder externe Standards bei der Messung verwendet werden.

### Beispiele:

### 1. Herstellung Aβ-Oligomerstandard

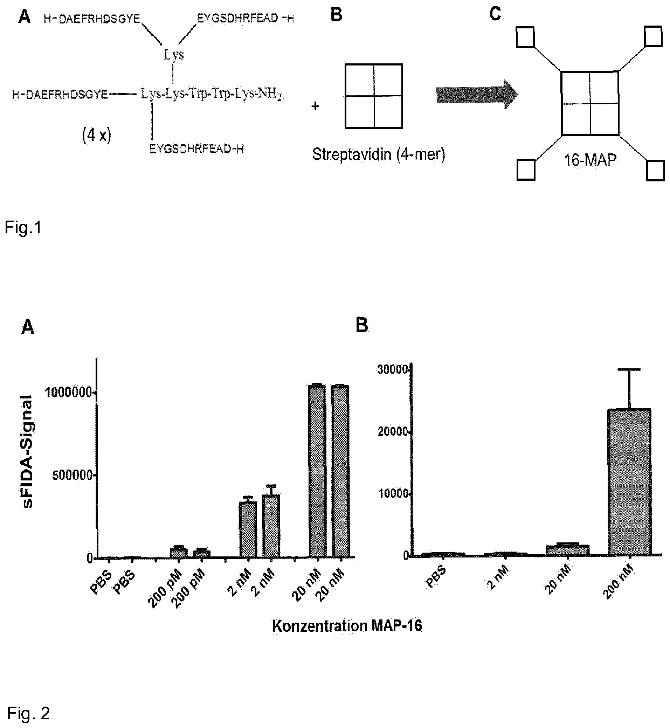

In einem Ausführungsbeispiel wurde ein Aβ-Oligomerstandard konstruiert, der 16 Epitope für N-terminal-bindende Aβ-Antikörper (Epitop entspricht Aβ₁₋₁₁, Sequenz: DAEFRHDSGYE, SEQ ID NO: 3), aufwies.
Zunächst wurde ein Multipel-Antigen-Peptid (MAP) synthetisiert, das aus vier N-terminalen Aβ-Epitopen Aβ1-11 bestand. Diese waren entsprechend Figur 1A an einen dreifach-Lysin-Kern gekoppelt, welcher zur genauen Bestimmung der MAP-Konzentration mittels UV/VIS-Spektroskopie zwei Tryptophane enthielt. Zusätzlich war N-terminal ein Biotin-Tag angefügt. Dieses diente der Kopplung von jeweils vier 4-MAP-Einheiten an ein Streptavidin-Tetramer, in Figur 1 unter B dargestellt. Nach Inkubation von 4-MAP und Streptavidin wurde 16-MAP gebildet, wie in Figur 1 unter C dargestellt. 16-MAP wurde durch Größenausschlusschromatographie von anderen Bestandteilen des Inkubationsansatzes getrennt.
Anschließend wurde MAP-16 seriell in PBS verdünnt und in den sFIDA-Test zur Detektion von Aβ-Oligomeren eingesetzt.

### 2. Detektion von Aβ-Oligomeren

### a. Vorbereitung Glasplatte

Mikrotiterplatten aus Glas wurden in einem Ultraschallbad über 15 Minuten gereinigt und anschließend mit einem Plasma-Cleaner für 10 min behandelt. Für die Aktivierung der Glasberfläche wurden die Wells in 5 M NaOH mindestens 3 Stunden inkubiert, mit Wasser gespült und dann im Stickstoff-Gasstrom getrocknet. Für die Beschichtung mit Dextran wurde die Glasoberfläche hydroxyliert und anschließend mit Aminogruppen aktiviert. Hierzu wurde die Glasplatten in einer Lösung von 5 M Ethanolamin in DMSO über Nacht inkubiert. Anschließend wurden die Glasplatten mit Wasser gespült und in einem Stickstoff-Gasstrom getrocknet. Carboxymethyl-Dextran (CMD) wurde in Wasser in einer Konzentration von 20 mg auf ml gelöst und mit N-Ethyl-N-(3-Dimethylaminopropyl) Carbodiimid (EDC), (200 mM) und N-Hydroxysuccinimid (NHS), (50 mM) gemischt. Nach einer Vorinkubation von 10 Minuten wurde die Lösung für weitere 2 Stunden bei Raumtemperatur inkubiert. Anschließend wurden die Glasplatten mit Wasser gewaschen.

### b. Immobilisierung von Antikörpern als Fängermoleküle auf dem beschichteten Glas

Eine zweite Aktivierung erfolgte mit einer Lösung von EDC/NHS (200 bzw. 50 mM) für 5 Minuten. Die Lösung der Antikörper wurde hinzu gegeben und für 2 Stunden bei 4°C inkubiert. Dadurch wurden die Antikörper kovalent an die mit CMD aktivierte Glasoberfläche gebunden. Um anschließend verbleibende aktive Carboxyl-Endgruppen auf dem CMD-Spacer zu deaktivieren, wurde mit 1 M-Ethanolamin in DMSO für 5 Minuten inkubiert. Das Glas wurde anschließend dreimal mit PBS gewaschen.

### c. Immobilisierung von MAP-16 auf dem vorbehandelten Glas

Die zu vermessende MAP-16-enthaltende Probe wurde 1 Stunde auf dem Glas inkubiert, anschließend dreimal mit TBST (0,1 %) (W/W), Tween-20 in TBS-Puffer, TBS: 50 nM Tris-HCI, 0.15 M NaCl, pH 7,4) gewaschen.

### d. Kennzeichnung der Sonden mit Fluoreszenzfarbstoff

Es wurden 6E10-Alexa-488-Antikörper und IC-16 Antikörper eingesetzt. Die IC16Antikörper wurden mit einen Kit (Fluoreszenzlabelling-KIT Alexa-647, Molecular Probes, Karlsruhe, Deutschland) gemäß den Angaben des Herstellers markiert. Die gekennzeichneten Antikörper wurden in PBS mit 2 mM Natriumazid bei 4°C im Dunkeln gelagert.

### e. Markierung der Aggregate mit den Sonden

Die Sonden wurden hinzugefügt und 1 Stunde bei Raumtemperatur inkubiert, anschließend fünfmal mit TBST und zweimal mit Wasser gewaschen.

### f. Detektion des Aggregatstandards

Die Messung erfolgte mit einem konfokalen Laser-Scanning-Mikroskop LSM 710 (Carl Zeiss, Jena, Germany). Das Mikroskop war mit einem Argon-Ionen-Laser und drei Helium-Neonlaser ausgestattet. Die Messungen erfolgten im Tile-Scan-Modus, bei dem benachbarte Flächen in einem Well gemessen und zu einem Bild zusammengesetzt werden. Jeder Tile-Scan enthielt 3 x 2 Einzelbilder, jedes Bild hatte eine Fläche von 213 x 213 µm.
Alternativ erfolgten die Messungen an einem TIRF-Mikroskop (TIRF = total internal reflexion), bestehend aus einem invertierten Mikroskop DMI 6000, einer Laserbox und einer Hamamatsu-EM-CCD C9100-Kamera. Im Tile-Scan-Modus wurden 3 x 3 Einzelbilder mit einer jeweiligen Größe von 109,9 x 109,9 µm.

Die Auswertung erfolgte mit der Software "Image J" ((http://rsbweb.nih.gov/ij/). Durch den Einsatz von unterschiedlichen Sonden konnte eine Kolokalisationsanalyse durchgeführt werden. Hierzu wurde zunächst ein cut-off -Wert, definiert durch eine Negativkontrolle ohne MAP-16, von den Intensitätswerten der einzelnen Pixel abgezogen. Anschließend wurde die Anzahl der kolokalisierten Pixel, deren Intensität größer als Null war, addiert.

Figur 2 zeigt die Ergebnisse der Messungen. Es ist deutlich zu erkennen, dass das sFIDA-Signal, d.h. die Menge der kolokalisierten Pixel, mit der Konzentration der MAP-16-Moleküle korreliert.

### Figurenbeschreibung

Figur 1: Konstruktion eines Aß-Oligomerstandards mit 16 Epitopen für N-terminal-bindende Aß-Antikörper, die den ersten 11 Aminosäuren von Aß entsprechen (Sequenz: DAEFRHDSGYE). A) 4-MAP wurde synthetisiert, bestehend aus 4 N-terminalen Aß-Epitopen1-11 gekoppelt an einen dreifach-Lysin-Kern, der zur Konzentrationsbestimmung mittels UV/VIS-Spektroskopie zwei Tryptophane enthielt. B und C) Zur Herstellung von 16-MAP wurden jeweils vier 4-MAP über ein Streptavidin-Teramer gekoppelt. MAP-16 wurde mittels Größenausschlusschromatographie von anderen Bestandteilen des Inkubationsansatzes getrennt.
Figur 2: sFIDA-Messungen von MAP-16 in verschiedenen Konzentrationen, verdünnt in PBS-Puffer. PBS-Puffer ohne MAP-16 diente als Negativkontrolle. A) Die Messungen wurden an einem Laserscanningmikroskop (Zeiss LSM 710) durchgeführt. B). Die Messungen wurden an einem TIRF-Mikroskop (Leica) durchgeführt.

## Patentansprüche

1. Standard zur Quantifizierung von pathogenen Aggregaten oder Oligomeren aus körpereigenen Proteinen, die eine Proteinaggregationserkrankung oder eine amyloide Degeneration oder Proteinfehlfaltungserkrankung kennzeichnen, **dadurch gekennzeichnet, dass** ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit jenen körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

2. Standard nach Anspruch 1 **dadurch gekennzeichnet, dass** dieser eine genau definierte Anzahl von Epitopen, die kovalent miteinader verknüpft sind, für die Bindung von Sonden besitzt.

3. Standard nach einem der vorangehenden Ansprüche enthaltend Epitope des A-beta-Peptids.

4. Standard nach Anspruch 3 enthaltend mindestens ein Epitop ausgewählt aus der Gruppe bestehend aus: A-Beta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5), pyroGluA-Beta 3 -11 (SEQ ID NO: 6), A-Beta 11 - 16 (SEQ ID NO: 7) und pyroGluA-Beta 11-16 (SEQ ID NO: 8).

5. Standard nach einem der vorangegangenen Ansprüche **dadurch gekennzeichnet, dass** er im Wäßrigen löslich ist.

6. Standard nach einem der vorangehenden Ansprüche enthaltend funktionelle Gruppen.

7. Standard nach einem der vorangehenden Ansprüche enthaltend mindestens ein Spacer-Molekül.

8. Standard nach einem der vorangehenden Ansprüche enthaltend Farbstoffe zur spektralphotometrischen Bestimmung und/oder aromatische Aminosäuren.

9. Standard nach einem der vorangehenden Ansprüche **dadurch gekennzeichnet, dass** die Polypeptidsequenzen nicht über eine Bindung zu einem Schwefelatom, nicht über eine Thioether-Bindung und/oder nicht über Cystein miteinander oder mit anderen Elementen der Standards verknüpft, insbesondere kovalent gebunden sind.

10. Standard nach einem der vorangehenden Ansprüche **dadurch gekennzeichnet, dass** die Polypeptidsequenzen in einer linearen, verzweigten oder cross-linked-Konformation miteinander verbunden sind, oder als Dendrimer vorliegen.

11. Dendrimere enthaltend Polypeptide, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung kennzeichnen, wobei die Polymere nicht aggregieren.

12. Dendrimere nach Anspruch 10 enthaltend mindestens ein Merkmal gemäß einem der Ansprüche 2 bis 9.

13. Verfahren zur Herstellung eines Standards nach einem der Ansprüche 1 bis 6 oder eines Dendrimers nach einem der Ansprüche 11 bis 12 **dadurch gekennzeichnet, dass** Peptidsynthese oder rekombinater Verfahren eingesetzt werden.

14. Verwendung eines Standards nach einem der Ansprüche 1 bis 9 oder eines Dendrimers nach einem der Ansprüche 10 bis 12 zur Quantifizierung von pathogenen Aggregaten oder Oligomeren aus körpereigenen Proteinen, die eine Proteinaggregationserkrankung kennzeichnen.

15. Verwendung nach Anspruch 14 zur Quantifizierung von A-beta-Oligomeren.

16. Verwendung nach Anspruch 14 **dadurch gekennzeichnet, dass** die Standards nach einem der Ansprüche 1 bis 9 oder die Dendrimere nach einem der Ansprüche 11 bis 12 für die Kalibrierung der Surface-FIDA-Methode, Elisa, Sandwich-Elisa oder FACS eingesetzt werden.

17. Verwendung nach Anspruch 14 enthaltend folgende Schritte:
- in einem ersten Schritt die Standards nach einem der Ansprüche 1 bis 9 oder die Dendrimere nach einem der Ansprüche 11 bis 12 mit Sonden markiert werden und die Anzahl der an die Standards oder Dendrimere gebundenen Sonden bestimmt wird,
- in einem zweiten Schritt pathogene Aggregaten oder Oligomere aus körpereigenen Proteinen, die eine Proteinaggregationserkrankung kennzeichnen, mit Sonden markiert werden, die Zahl der an jeweils ein pathogenes Aggregat oder Oligomer bindende Sonden bestimmt wird,
- in einem dritten Schritt die Zahl der an jeweils ein Standard oder Dendrimer bindende Sonden aus Schritt 1 mit der aus Schritt 2 verglichen wird, und
- in einem vierten Schritt dadurch die Zahl und die Größe der Oligomere aus der Körperflüssigkeit bestimmt wird.

18. Kit enthaltend mindestens ein Standards nach einem der Ansprüche 1 bis 9 oder mindestens ein Dendrimers nach einem der Ansprüche 11 bis 12 zur Quantifizierung von pathogenen Aggregaten oder Oligomeren aus körpereigenen Proteinen, die eine Proteinaggregationserkrankung kennzeichnen.

19. Verfahren zur Quantifizierung von pathogenen Aggregaten oder Oligomeren aus körpereigenen Proteinen, die eine Proteinaggregationserkrankung oder eine amyloide Degeneration oder Proteinfehlfaltungserkrankung kennzeichnen, bei dem ein Polymer als Standard eingesetzt wird, das aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit jenen körpereigenen Proteinen aufweisen, die eine Proteinaggregationserkrankung oder eine amyloide Degeneration oder Proteinfehlfaltungserkrankung kennzeichnen, wobei die Polymere nicht aggregieren.


SEQUENCE LISTING

<110>  Forschungszentrum Jülich

<120>  Standard zur Quantifizierung von pathogenen Aggregaten  aus
       körpereigenen Proteinen

<130>  FZJ 1101 PCT

<160>  8

<170>  PatentIn version 3.5

<210>  1
<211>  43
<212>  PRT
<213>  human

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala Thr
        35                  40

<210>  2
<211>  8
<212>  PRT
<213>  Artificial Sequence

<220>
<223>  Polypeptid

<400>  2

Asp Ala Glu Phe Arg His Asp Ser
1               5

<210>  3
<211>  11
<212>  PRT
<213>  Artificial Sequence

<220>
<223>  Polypeptid

<400>  3

Asp Ala Glu Phe Arg His Asp Ser Gly Tyr Glu
1               5                   10

<210>  4
<211>  16
<212>  PRT

<213>  Artificial Sequence

<220>
<223>  Polypeptid

<400>  4

Asp Ala Glu Phe Arg His Asp Ser Gly Tyr Glu Val His His Gln Lys
1               5                   10                  15


<210>  5
<211>  9
<212>  PRT
<213>  Artificial Sequence

<220>
<223>  Polypeptid

<400>  5

Glu Phe Arg His Asp Ser Gly Tyr Glu
1               5


<210>  6
<211>  9
<212>  PRT
<213>  Artificial Sequence

<220>
<223>  Polypeptid


<220>
<221>  MISC_FEATURE
<222>  (1)..(1)
<223>  pyroglutamate

<400>  6

Glu Phe Arg His Asp Ser Gly Tyr Glu
1               5


<210>  7
<211>  6
<212>  PRT
<213>  Artificial Sequence

<220>
<223>  Polypeptid

<400>  7

Glu Val His His Gln Lys
1               5


<210>  8
<211>  6
<212>  PRT
<213>  Artificial Sequence

<220>
<223>  Polypeptid

<220>
<221>  MISC_FEATURE
<222>  (1)..(1)
<223>  pyroglutamat

<400>  8

Glu Val His His Gln Lys
1               5